# EUROPEAN PATENT SPECIFICATION

(11) **EP 3 583 942 B1**
(45) Date of publication and mention of the grant of the patent: **10.01.2024**
(21) Application number: 19177784.6
(22) Date of filing: 31.05.2019
(51) Int. Cl.: A61K 31/352, A61P 25/28

(54) **GENISTEIN FOR USE IN A METHOD OF TREATMENT OF ALZHEIMER'S DISEASE AS STIMULATOR OF THE AUTOPHAGY PROCESS IN A PROCESS OF LYSOSOMAL SYSTEM ACTIVATION**
GENISTEIN ZUR VERWENDUNG IN EINER METHODE ZUR BEHANDLUNG VON MORBUS ALZHEIMER ALS EIN STIMULANS DES AUTOPHAGIE PROZESSES IN EINEM PROZESS DER LYSOSOMALEN SYSTEMAKTIVIERUNG
GENISTEIN POUR SON UTILISATION DANS UNE MÉTHODE DU TRAITEMENT DE LA MALADIE D'ALZHEIMER COMME STIMULATEUR D'UN PROCÉS DE LA AUTOPHAGIE DANS UN PROCÉDÉ D'ACTIVATION DU SYSTÈME LYSOSOMALE

(30) Priority: 06.06.2018 PL 42583218
(43) Date of publication of application: 25.12.2019
(73) Proprietor: Uniwersytet Gdanski, 80-309 Gdansk (PL)
(72) Inventor: Wegrzyn, Grzegorz, 80299 Gdansk (PL); Pierzynowska, Karolina, 80-281 Gdansk (PL); Podlacha, Magdalena, 80-363 Gdansk (PL); Myslinska, Dorota, 83-000 Bedzieszyn (PL); Majkutewicz, Irena, 83-110 Tczew (PL); Gaffke, Lidia, 84-200 Wejherowo (PL)
(74) Representative: Pawlowska, Justyna

(56) References cited:
- US-B1- 9 744 204
- KAROLINA PIERZYNOWSKA ET AL: "Correction of Huntington's Disease Phenotype by Genistein-Induced Autophagy in the Cellular Model", NEUROMOLECULAR MEDICINE, vol. 20, no. 1, 12 February 2018 (2018-02-12), pages 112-123, XP55638780, US ISSN: 1535-1084, DOI: 10.1007/s12017-018-8482-1
- PIERZYNOWSKA KAROLINA ET AL: "Stimulation of autophagy by genistein reduces levels of mutant huntingtin in cellular models of Huntington disease", MOLECULAR GENETICS AND METABOLISM, vol. 120, no. 1-2, January 2017 (2017-01), page S107, XP009516999, & 13TH ANNUAL RESEARCH MEETING ON WE'RE ORGANIZING RESEARCH FOR LYSOSOMAL DISEASES (WORLD); SAN DIEGO, CA, USA; FEBRUARY 13 -17, 2017
- MARYAM BAGHERI ET AL: "Genistein ameliorates learning and memory deficits in amyloidrat model of Alzheimers disease", NEUROBIOLOGY OF LEARNING AND MEMORY, SAN DIEGO, US, vol. 95, no. 3, 1 December 2010 (2010-12-01), pages 270-276, XP028367094, ISSN: 1074-7427, DOI: 10.1016/J.NLM.2010.12.001 [retrieved on 2010-12-07]
- PIERZYNOWSKA KAROLINA ET AL: "Autophagy-dependent mechanism of genistein-mediated elimination of behavioral and biochemical defects in the rat model of sporadic Alzheimer's disease", NEUROPHARMACOLOGY, vol. 148, 31 January 2019 (2019-01-31), pages 332-346, XP085640822, ISSN: 0028-3908, DOI: 10.1016/J.NEUROPHARM.2019.01.030

## Description

The present invention relates to medical use of genistein for use in the therapy of Alzheimer's disease.. The medical use according to the invention concerns the use of genistein to activate the autophagy process, while the mechanisms of action in the therapeutic use deals with degradation pathogenic proteins located in a brain in Alzheimer's disease - beta-amyloid and P-tau protein, hyperphosphorylated form of tau protein.

Alzheimer's disease (AD) is a disease of elderly people, while its prevalence has been increasing due to senescence of the society. A lack of pharmaceuticals for this disease is a serious problem which is expected to increase within next decades. Currently, the number of patients suffering from AD exceeds 50 million in the world, including over 300,000 in Poland, while it is worth emphasized that diagnosis of the disease is difficult due to attributing the first symptoms of the disease to senescence.

They are two types of AD, familial (fAD), which is diagnosed when in patient's family there are at least 2 cases of the disease (approximately 15-40% of AD population), and sporadic AD (sAD; 60-85% of AD population) which is more frequent and does not have a genetic basis. While fAD is inherited in the autosomal dominant manner, sAD is a multifactorial disease. In the brain of AD patients, there are aggregates of amyloid plaques and neurofibrillary tangles which negatively influence on functions of neurons and impair signal transduction between neural cells, leading to neurodegeneration of some brain regions (particularly cortex, hippocamp and amygdala). The disease is caused by accumulation of toxin beta-amyloid (βA), forming senile plaques, and hyperphosphorylated tau protein (P-tau), being the major component of neurofibrillary tangles in the central nervous system. The reason for accumulation of these two proteins is so far unsolved problem. In fAD, mutations in genes coding for proteins involved in formation of (βA) from its precursor were found, however, in sAD causes remain totally unknown.

The main symptoms of AD are disorders of abstractive thinking and semantic memory (early stages), language problems, long-time memory deficiency and personality changes (late stages). Patients are excluded from social and familial life. Their life functions are systematically lost which leads to death during 7 years from diagnosis, on average.

Despite many attempts to find a cure, no effective therapy for AD has been developed so far. One of molecular therapeutic strategies was inhibition of secretases involved in the transitions of the βA precursor and kinases which phosphorylate the tau protein which was expected to prevent formation of βA and P-tau. However, the inhibitor employed in the studies caused severe adverse effects in experiments on animal models of the disease, apparently due to their low specificity. Another strategy was the use of specific antibodies, binding and inactivating βA, however, this strategy was not successful, similarly to attempts of increasing neurons' survival by blocking biochemical pathways leading to apoptosis. The strategy of inhibition of synthesis of the amyloid precursor and tau protein was also not effective, as these proteins play important functions in the organism when occurring in a proper form.

From the patent description PL188481, the use of 2-phenyl-1,2-benzoisoselenasol-3(2H)-one for production of potential anti AD drug is known.

From the patent application WO95/04538, the use of 4'-iod-4'-deoxydoxorubicin for production of potential anti AD drug is also known.

In further patent application EP2075007 there is disclosed a peptide conjugated with a protein which can act as an immunogenic agent to produce antibodies causing reduction of amyloid plaques accumulation, in order to obtain anti AD drug.

From patent application EP2157973, donepesil chloride is known as a potentail anti AD drug.

There are many activities of genisten, especially as an anti-inflammatory agent.

Patent application WO2018049141 describes various compounds, including genistein, acting as phytoestrogens or modulators of immunological response, which are anti-inflammatory agents.

In Pierzynowska et al. Correction of Huntington's Disease Phenotype by Genistein-Induced Autophagy in the Cellular Model, Neuromolecular Med. 2018 Mar;20(1):112-123, there is description of studies about effects of genistein on a Huntington's disease using cellular model consisting of HEK-293 cells transfected with a plasmid bearing mutated HTT gene. Both level of mutated huntingtin and number of aggregates were significantly decreased in genistein-treated cell model. On the other hand Alzheimer's disease is a multifactor disease, caused by various agents and conditions, and the crucial changes are formation of beta-amyloid plaques and hyperphosphorylated tau protein (P-tau) fibers. When the model of the disease does not reflect the real patomechanism it is not possible to predict if genistein can be effective also in other diseases.

In Pierzynowska et al. Stimulation of autophagy by genistein reduces levels of mutant huntingtin in cellular models of Huntington disease January 2017Molecular Genetics and Metabolism 120(1-2) it was disclosed genistein (5, 7-dihydroxy-3- (4-hydroxyphenyl)-4H-1-benzopyran-4-one) with a number of biological activities. Three is hypothesis about its role in treatment of patients suffering from Huntington's disease.Presence of genistein in culture medium resulted in a significant decrease in the number of mutant huntingtin aggregates, as well as in the reduction of their volume. Biochemical analyses indicated that the amount of mutant huntingtin decreased considerably in genistein-treated cells relative to controls. Under these conditions, survival of such cells increased considerably. Genistein was able to stimulate the process of autophagy, as demonstrated by an increased level of the LC3-II protein in cells in the presence of this isoflavone in the culture medium. In the document D2 there are general discussions and speculations on autophagy in neurodegenerative diseases. On the other hand efficiency can be very different in various diseases.

The document: Bagheri et. al. Genistein ameliorates learning and memory deficits in amyloid β(1-40) rat model of Alzheimer's disease, Comparative Study Neurobiol Learn Mem. 2011 Mar;95(3):270-6 deals with Alzheimer's disease. In disclosed study effects of genistein in oxidative stress markers. They showed an elevation of malondialdehyde and nitrite content, and a reduction of superoxide dismutase activity in hippocampal tissue of the rats. The mode used by authors of is not a full-disease model of Alzheimer disease.

In the treatment of AD, various compounds are known as active agents, including rivastigmine, donepesil, galantamine and memantine, which action is based on keeping a relatively high level of acetylcholine, predominantly due to inhibition of the enzyme inhibiting its degradation, considering that decreased levels of acetylcholine is negatively correlated with cognitive processes and long-term memory. However, all the compounds listed above cause various adverse effects, from mild, like sleep problems, headache, impairment of digestion, to severe, like muscular stiffness, hallucination, psychotic events, bradycardia, neurovascular disorders.

Therefore, currently one of the most promising therapeutic approaches is enhanced degradation of toxin forms of aggregated βA and P-tau. Although many activators of protein degradation pathways were already tested, no compound was found which could degrade accumulated proteins and be safe in a long-term therapeutic use.

According to the present invention, another therapeutic activity of genistein is proposed, as an autophagy stimulatorwhich is involved in degradation of abnormal cellular structures and macromolecules - toxic proteins accumulating in the brain in AD.

Inventors of the present invention had been investigating the possibility of the use of genistein as a drug for treatment of AD extensively. They found that this compound has excellent effects in AD treatment, leading to a decrease in levels of βA and P-tau due to stimulation of autophagy, due to therapeutic activity which is different than those already known in the state of the art.

The present invention provides genistein, one of isoflavones, formula 1, for use in a method of treatment of ADat a dose of 150 mg/kg/day as a stimulator of autophagy process, characterized in that, the genistein is used at a dose 150 mg/kg/da. The invention leads to a decrease or even total reduction of level of toxic proteins which cause AD - βA and P-tau. Preferably genistein for use in the treatment of Alzheimer's disease is administered orally and is administered in form of as a powder or water suspension.

The medical use of genistein, according to the invention, is based on the used therapeutic activity - stimulation of autophagy, leading to destruction of toxic protein.

The effects of genistein were confirmed in both *in vitro* studies and experiments with the animal model of AD, in which increased accumulation of the toxic proteins occurs. The reduction of the disease symptoms were indicated in tests confirming the disease detection, especially in behavioral tests.

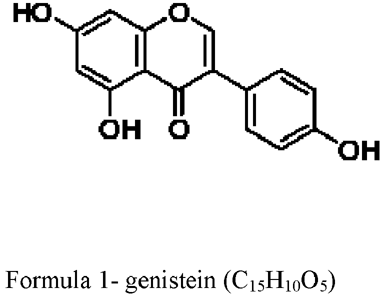

According to the experiments with the use of genistein according to the invention, it was demonstrated that therapeutic activity of this compound is based on induction of one of the protein degradation processes - the autophagy, contrary to effects on apoptosis or antioxidant activity. Autophagy inductionleads to removal of the cause of the disease rather than only to attenuation of secondary effects of AD.

In experiments performed with the animal model of AD, induced with injection of the toxin - streptozotocin, it was demonstrated that behavior of animals was improved after treatment with genistein. A decrease in the levels of pathogenic proteins, βA and P-tau, was observed in brains of animals treated with genistein.. This provides evidence for the autophagy stimulation-dependent mechanisms of therapeutic effects of genistein in AD.

The object of the invention is genistein for use in a method of treatment of AD, wherein the genistein acts as a therapeutic agent through stimulation of autophagyleading to degradation of βA and P-tau.

According to the invention the genistein is used at a dose 150 mg/kg/day.

In a preferred embodiment of the use, genistein is administered orally, preferably as a powder or water suspension.

The invention is presented in details in examples and figures, which demonstrates experiments confirming the medical use of genistein according to its therapeutic activity.

Description of the figures:

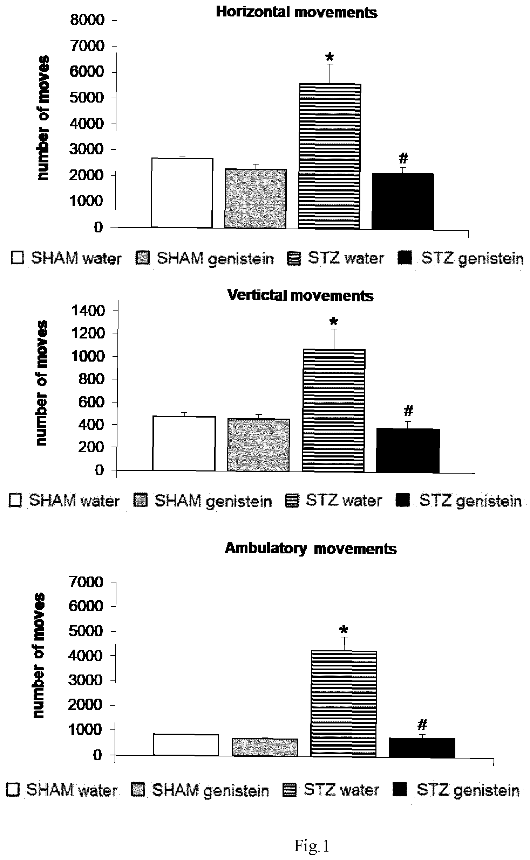

Fig.1 - The figure presents determination of motoric activity of animals, particularly number of horizontal, vertical and ambulatory movements. The presented results indicate that animals with induced Alzheimer disease (AD) (the STZ + CTR group) are characterized with significant motoric overactivity which is expressed by increased number of horizontal, vertical and ambulatory movements (the values are 2-3-fold higher relative to the control group, VEH+CTR). Number of each kind of movements in AD animals treated with genistein is comparable to that in the control group. Asterisk indicate statistical significance of the results in the indicated group relative to the control group (VEH+CTR) at p<0.05, or p<0.01 for two asterisks.

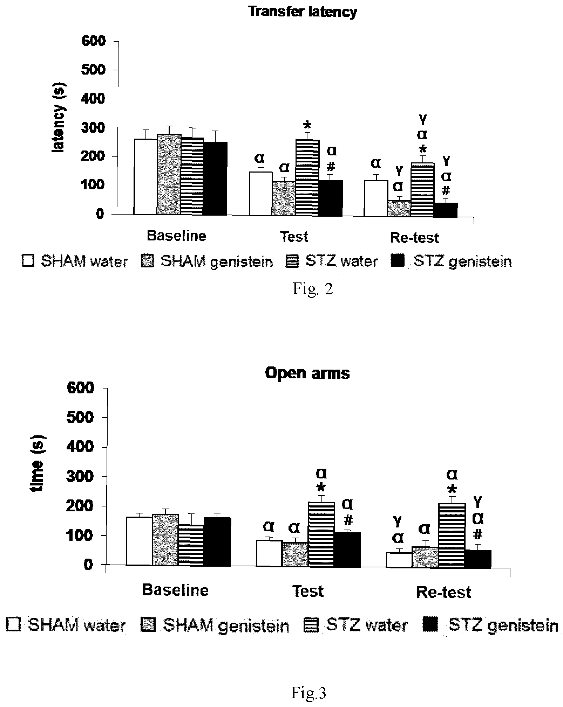

Fig.2 - The figure presents results of estimation of memory in the elevated plus test, particularly the time necessary to reach the closed arm from the open arm (the initial point of the test). Healthy rats are able to reach the closed arm in shorter and shorter time after each repetition of the test (test + re-test). The diagram indicates the baseline results which are similar in each animal group, while even during the fist test, the time to reach the closed arm was shorter in healthy rats and AD rats treated with genistein, relative to AD rats. The time i n the group of AD rats (STZ+CTR), the time remained similar to the baseline after each repeat in comparison to baseline results. In the re-test, the time was shorten in the control group and AD rats treated with genistein, while being prolonged in untreated AD rats. Three asterisks indicate statistically significant difference (p<0.001) in the first measurement relative to the control group (VEH+CTR), while three gamma symbols indicate statistically significant difference (p<0.001) in the second measurement (re-test) relative to the control group (VEH+CTR). Three alpha symbols indicate statistically significant difference (p<0.001) relative to baseline.
Fig.3 - The figure presents results of estimation of memory in the elevated plus test, particularly time spent in the open arms. Healthy rats, beacuse of avoiding heigh and open field, should spend as short time in the open arms as possible, and this time should be shortened in each subsequent measurement (test and re-test). The results indicate baseline values, which are similar in each group, while in the first test, time spent in open arms is shorter for healthy rats and AD rats treated with genistein. In untreated AD rats (STZ+CTR), the time remains similar at baseline and in the test. In the re-test, time spent in open arms is even more shorter for healthy rats and AD rats treated with genistein, while remaining similar in untreated AD rats (STZ+CTR). The gamma symbol (in re-test) indicates statistacally significant differences between particular group and the VEH CTR group (two symbols for p<0.01, three symbols for p<0.001); three Symbol gamma (w teście re-test) oznacza istotność statystyczn 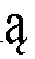 danej grupy w stosunku do grupy VEH CTR podczas pomiaru "test" (dwa symbole - p<0,01; trzy symbole - p<0,001); Three alpha symbols indicate statistically significant difference (p<0.001) relative to baseline.

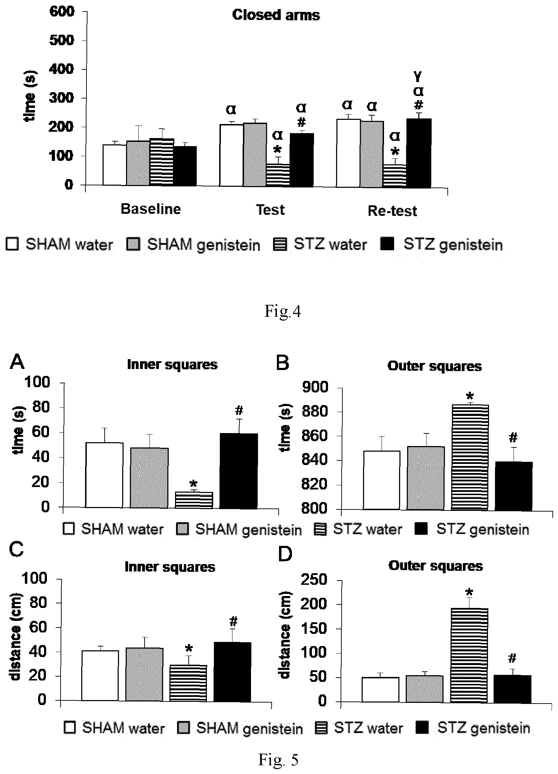

Fig.4 - The figure presents results of estimation of memory in the elevated plus test, particularly time spent in the closed arms (in opposed to Fig.3). Healthy rats, beacuse of avoiding height and open field, should spend as long time in the closed arms as possible, and this time should be prolonged in each subsequent measurement (test and re-test). The results indicate baseline values, which are similar in each group, while in the first test of measurement, time spent in closed arms is longer for healthy rats and AD rats treated with genistein. In untreated AD rats (STZ+CTR), the time remains similar at baseline and in the test. In the re-test, time spent in closed arms is even longer for healthy rats and AD rats treated with genistein, while remaining similar in untreated AD rats (STZ+CTR) (short time). The description of the used symbols are as in Fig. 3.
Fig.5 - The figure presents results of estimation of animal behavior in the open field, particularly time spent in central and peripheral squares, as well as distance (in cm) traveled in these two types of squares. This test allows to assess animal preference for the place occupied in the field during 20 min session, which is a measure of locomotor activity and anxious behavior. The results indicated that animals with induced AD (STZ CTR) spent a relatively short time in central squares and long time in peripheral squares in comparison to the control group (VEH CTR). AD rats treated with genistein had results identical to control (healthy) animals. Similar results were obtained for measurement of the distance traveled by rats (the measure of mobility). The distance traveled by AD rats in shorten in central squares relative to control animals, while it is longer in peripheral ones. Genistein is able to abolish the changes caused by the disease also in this parameter. Two asterisks indicate statistically significant differences relative to the control group (VEH CTR) at p<0.01.

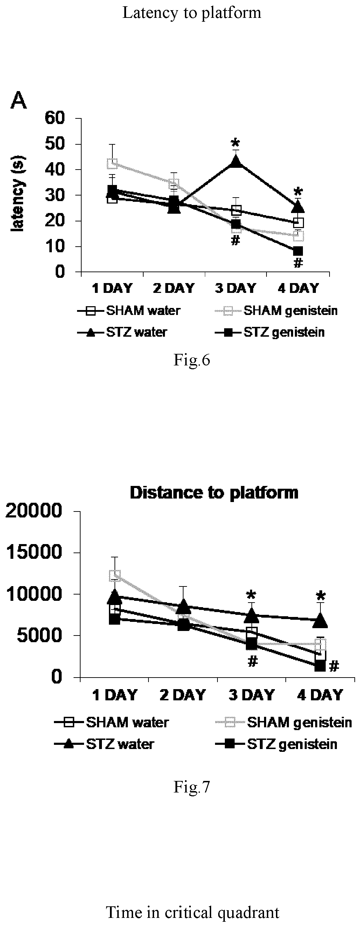

Fig.6 - The figure presents results of memory estimation in the Morris water maze test, particularly the time necessary to find a platform by animals. The platform is invisible for animals, and the experiment in conducted for 4 days in which rats should find the platform quicker and quicker every day (which is the measure of the reference memory). The figure shows the time required to find the platform in each group of animals. The results indicated that in control (healthy) animals, as well as in AD rats treated with genistein, the time is shorter and shorter every day, i.e. the rats found the platform quicker and quicker. However, the time in AD rats (STZ CTR) remained random every day. Three asterisks indicate statistically significant differences relative to the STZ-genistein group (p<0.001).
Fig.7 - The figure presents results of memory estimation in the Morris water maze, particularly distance traveled by animals to reach the platform. The measurement indicates if animals went straight to the platform of were looking for it randomly. The distance is shorten (distance dropped) every day of experiment in all groups, however, it is worth noting that in healthy animals and genistein-treated AD rats, the value dropped by 70%, while in untreated AD rats only by 30%. Asterisk indicates statisitically siganificant differences relative to the STZ genistein group.

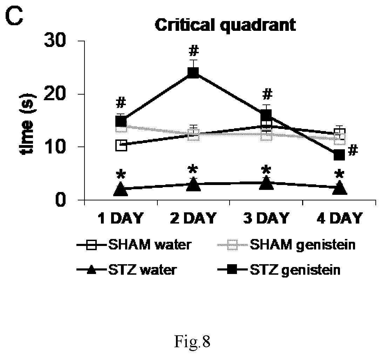

Fig.8 - The figure presents results of memory estimation in the Morris water maze, particularly the time spent in the quadrant of the pool in which the platform is located. The measurement indicates if animals can remember the region of the location of the platform. The results indicated that control animals and AD rats treated with genistein spent in the platform quadrant more time than untreated AD rats (STZ CTR). Three asterisks indicate statistically significant differences relative to the STZ genistein group (p<0.001).

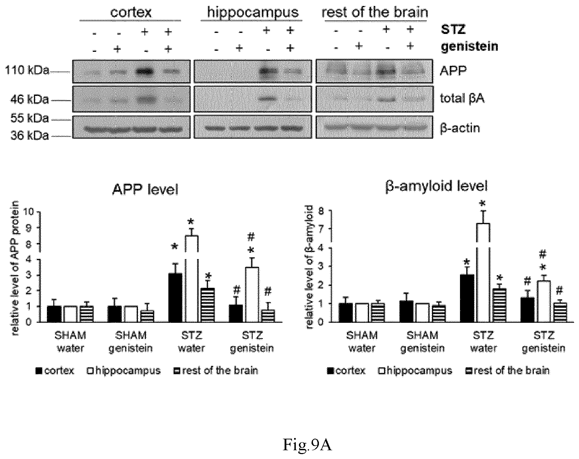

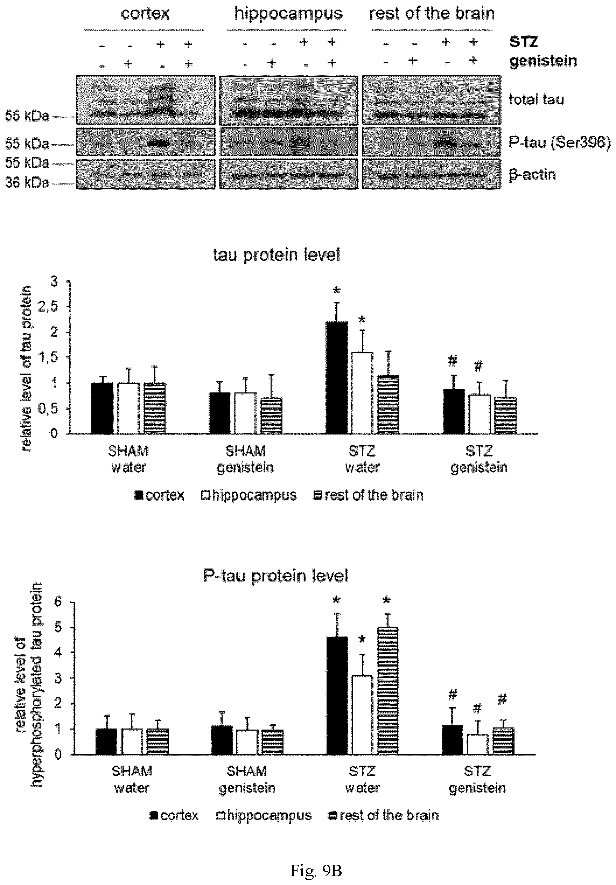

Fig.9A and Fig.9B - The figure presents results of biochemical analyses, particularly estimation of levels of proteins causing Alzheimer disease (beta-amyloid, Fig.9A, and P-tau, Fig.9B) in the rat brain. Levels of these proteins in control groups (healthy animals treated and untreated with genistein) are not changed. However, their levels are elevated significantly in AD rats, being about 3-times more abundant than in controls. In AD animals treated with genistein, levels of these proteins is normalized to the levels found in healthy rats. Asterisk indicates statistically significant differences relative to the control group (VEH water) (p<0.05), while # indicates statistically significant differences relative to the STZ water group (p<0.05).

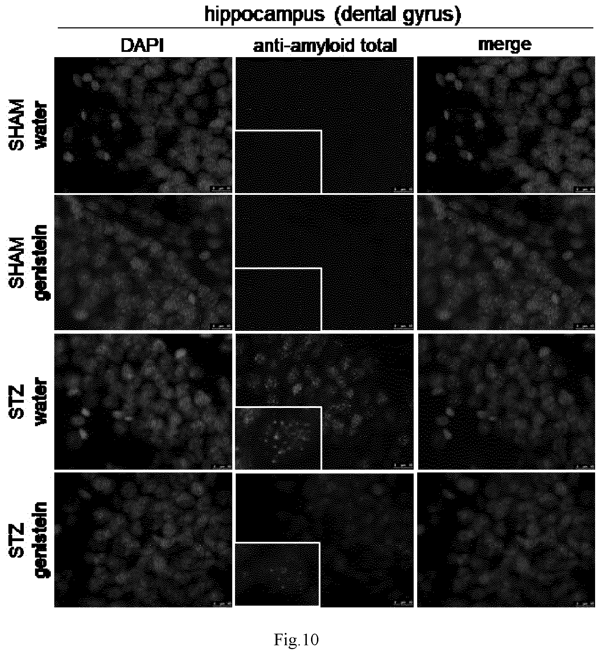

Fig. 10 - The figure presents visualization of beta-amyloid aggregates in neurons of the hippocampus (the structure responsible for memory). The microphotographs indicate that no beta-amyloid plaques could be found in the control group, while in AD animals, they are clearly visible in large amount. Genistein reduces amount of beta-amyloid causing disappearance of the aggregates in some neurons, facilitating their normal function.

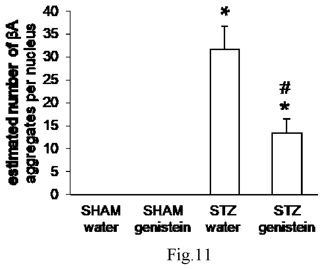

Fig. 11 - The figure represents quantitative analysis of the results presented in Fig. 10. Beta-amyloid aggregates are not detectable in control (healthy) animals, while in AD rats occur in the mean number of 32 per cell. Genistein reduces this number to the level of 12 per cell. Three asterisks and three alpha symbols indicate statistically significant differences relative to control (VEH water) (p<0.001), while three # symbols indicate statistically significant differences relative to the STZ - water group (p<0.5)

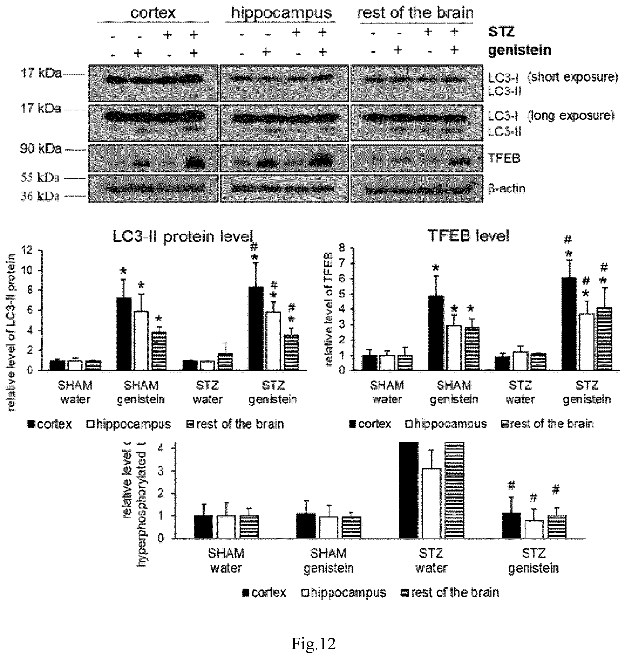

Fig. 12 - The figure presents autophagy induction by genistein in the brains of AD rats. Levels of LC3-II and TFEB proteins in extracts from particular parts of the brain (cortex, hippocampus, the test of the brain) were determined by Western-blotting. The brains were isolated from rats subjected to intraventricular injection of streptozotocin (STZ) or buffer (SHAM) treated with either genistein (150 mg/kg/day) or water for 30 days. Representative blots are shown in panel A, while their quantitative analysis is demonstrated in panels B and C. Number of lysosomes was determined by fluorescence microscopy (panels D-F) of hippocampus (region CA1) of rats subjected to intraventricular injection of streptozotocin (STZ) or buffer (SHAM), treated with either genistein (150 mg/kg/day) or water for 90 days. Representative microphotography is shown in panel D, and quantitative analysis is demonstrated in panels E and F. In panels B, C, E and F, results are shown as mean value ± SD. Symbols indicate statistically significant differences (p<0.05) between particular groups and water-SHAM (*). One way ANOVA with Tukey post hoc test was used.

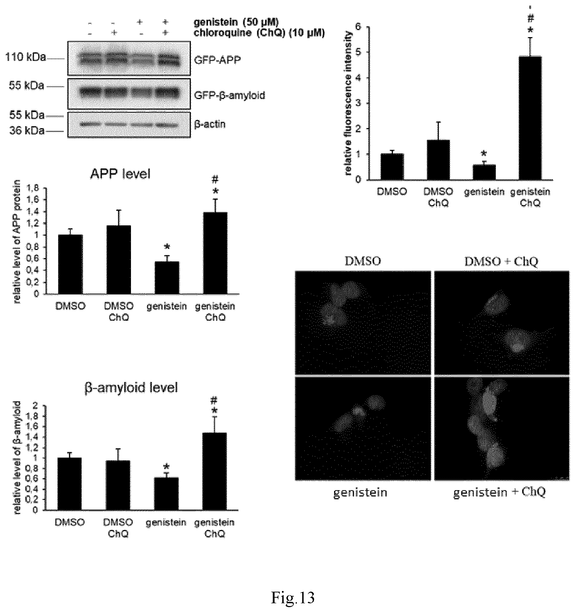

Fig. 13 - The figure presents inhibition of genistein-mediated decreasing of levels of amyloid precursor (APP protein) and beta-amlyloid (βA), due to chloroquine-mediated inhibtion of autophagy. Levels of APP and βA in HEK-293 cells transfected with plasmid pEGFP-n1-APP, overexpressing the gene coding for the fusion protein APP-EGFP, were measured by Western-blotting (panels A-C) and fluorescence microscopy (panels D, E). Following compounds were added to various cell cultures: DMSO (control), 10 µM chloroquine (ChQ; autophagy inhibitor), 50 µM genistein or mixture of 10 µM chloroquine and 50 µM genistein. Representative blots are shown in panel A, and quantitative analysis is shown in panels B and C. Representative microphotographs are shown in panel D, and quantitative analysis (in which only transfected cells were considered) is presented in panel E. In panels B, C and E, results are shown as mean values ± SD. Symbols indicate statistically significant differences (p<0.05) between particular results and DMSO (*), between results for genistein and the genistein+ChQ mixture (#) or between particular groups and DMSO+ChQ (γ). One way ANOVA with Tukey post hoc test was used.

### Examples

The aim of the study was to determine efficiency of genistein action as a potential drug for Alzheimer disease (AD), including:
- Determination of efficiency of degradation of two proteins causing AD, βA (beta-amyloid) and P-tau (hyperphosphorylated form of the tau protein), by genistein
- Estimation of effects of genistein on memory deficit and cognitive impairment
- Determination of therapeutic activity of genistein, as in the subject of the invention.

### Example 1

A streptozotocin-induced rat model of AD was employed. Streptozotocin (STZ) is a derivative of glucosamine and nitrosourea, produced by *Streptomyces achromogenes.* When administered intraventricularly to rats, it enters the cells through the GLUT-2 receptor, causing production of reactive oxygen species, inflammatory response (such reaction were also observed in human AD disease) and formation of βA plaques and neurofibrillary tangles composed of the P-tau protein. As a result, cholinergic transmission is impaired which leads to neurodegeneration, mainly in the hippocampus, causing sever memory deficits.

Following surgery, including injection, were divided for 4 groups, indicated as:
1) VEH WATER group: with injection of the solvent for STZ, which were treated with water (healthy animals);
2) VEH GENISTEIN group: with injection of the solvent for STZ, which were treated with genistein at the dose of 150 mg/kg/day (healthy animals treated with genistein);
3) STZ WATER group: with injection of STZ, which were treated with water (untreated AD animals);
4) STZ GENISTEIN group: with injection of STZ, which were treated with genistein at the dose of 150 mg/kg/day (AD animals treated with genistein).

Genistein was administered orally.

After one month from the onset of genistein/water administration, a series of behavioral tests was conducted, including:
a) Locomotor activity measurement, performed in actometers;
b) Elevated plus maze test;
c) Monitoring of the natural behavior of animals in the open field - the open field test;
d) Memory assessment in the Morris water maze test.

Then, after 3 months from the onset of genistein/water administration, rats were sacrificed, the brain tissues were withdrawn and subjected to biochemical analyses in order to determine levels of toxin proteins which cause AD.

Genistein was used in a free form, as a product of chemical synthesis, in the form of a white powder. For oral administration at the final dose of 150 mg/kg/day, this compound was prepared as a suspension in water.

### Ad.a) Locomotor activity measurement

This test was performed for an analysis of locomotor activity of rats in a new environment. The environment consists of a plastic box (size: 43 x 43 x 20 cm), being a part of the actometer, an equipment used for quantitative determination of horizontal, vertical and ambulatory movements. The locomotor activity of all groups of animals was assessed always at the same time during the day, and the duration was 2 h (Fig. 1).

Interpretation: AD animals are characterized by high locomotor overactivity, thus, they are hyperactive. This feature is a symptom often observed in animal models of neurodegenerative diseases. Genistein administration resulted in complete correction of hyperactivity of AD animals, so that these animals were identical to healthy ones in the number of all kinds of movements.

### b) Assessment of memory and stress level in the elevated plus maze test

This test is based on the use of a maze, in the cruciform shape, mounted at the height of 50 cm over the ground, with two open a and two closed arms. Healthy rats, put at the open arm, in which the height is a stressor, should move to the closed arms and stay there till the end of experiment. Rats with neurodegenerative disease spend time equally (randomly) in both open and closed arms. Following parameters were measured: time for movement from open to closed arm, time spent in open/closed arms during the 5-min experiment. The measurements were repeated after 10 min interval (to assess working memory), in order to assess if the time for movement from open to closed arm shortens (Figs. 2-4).

Interpretation of Figures 2-4: Latency (time) of the movement from open to closed arms during the test and re-test, as well long time spent in closed arms and short time sent in open arms are indicators of memory of animals (reflecting ability to escape from stressing conditions). In the group of control rats (healthy groupe) and AD rats treated with genistein, the time of movement to the closed arm has shortened in each of the measurement time (test and reitest) , indicating well memory and ability to learn in these groups of animals. This is corroborated by time spent in closed and open arms. On the other hand, in AD animals no quick movement to the closed arm could be observed, nor short term spent in open arms and long term spent in closed arms, indicating that these animals are not able to remember the possibility to find a save place in the repeated experiment. Genistein-treated animals reveal the same level of memory as healthy animals.

### c) Open field test

This test is used to assess anxiety behavior in rats. The field is divided into 25 squares. Time spent in central and peripheral squares depends on individual preferences of rats and their anxiety behavior. The experiments were performed for 20 minutes in the case of each group of animals (Fig. 5).

Interpretation: Rats with AD are characterized by increased anxiety and uncertain behavior, which is expressed as shorten time of occupancy of central squares, and longer time spent in peripheral squares. Similar results were obtained in measurement of traveled distance which indicated disorientation in the field. Treatment with genistein caused an increase in the frequency of entering central squares by AD rats, with exploration of the field and lowered anxiety, like in the case of healthy rats.

### d) Morrisa water maze test

This test is one of the most frequently used tests for assessment of memory in animals. A pool of 150 cm diameter is filled with water at room temperature, and it is divided for 4 quadrants. In the center of one of them, a platform is mounted about 1 cm below the water surface. The test was preceded by a pre-test, in which the platform is mounted over the water surface. The experiment was conducted during 4 days, with 4 trials daily, including 10 min breaks. In a single experiment, the time is measured from the putting of a rat into the pool, to finding the platform, but not longer than 120 sec. Rats which cannot find the platform are transferred to it, and kept for 10 sec. Following parameters were measured: 1) time to find the platform, 2) time spent in the quadrant with the platform, 3) number of entrances to the quadrant with the platform, 4) distance travelled by a rat to find the platform. The shortening of the time to find the platform in subsequent days of the experiment is the measure of the long-term spatial memory. Healthy rats should find the platform quicker and quicker every next day, as well as they should spent longer time in the quadrant with the platform, and the distance travelled should be shorter and shorter. In contrast, rats with neurodegenerative disorders are characterized by random finding the platform every day (Fig. 6-8).

Interpretation of Figures 6, 7 and 8: Results of these tests indicate severe memory deficiency in rats with AD. These animals did not find the platform in shorter time every day, distance travelled for finding the platform did not differ significantly in subsequent days (dropped only in 30%), and time spent in the quadrant with the platform was short, indicating that the animals did not look for the platform in the proper region. On the other hand, both healthy rats and AD rats treated with genistein found the platform quicker and quicker every day, which indicates their ability to learn and well-developed memory. Moreover, distance travelled by these animals shortened by 70%, which shows that rats went directly to the platform as they learned about its location, and in addition, time spent in the quadrant with the platform was long, indicating that even if they failed to find the platform immediately, they remembered where it should be located. These results indicate AD rats treated with genistein were indistinguishable from healthy ones in this memory test.

### Conclusions:

All results of the behavioral tests indicated full correction of AD symptoms in rats, so these animals were indistinguishable from healthy rats.

### Example 2

Testing genistein activity according to its use.

Biochemical tests: Determination of levels of beta amyloid (βA) and its precursor (APP), and the P-tau protein in the brains of rats (beta-amyloid and P-tau are proteins causing AD).

The brains (hippocampus, cortex, and the rest) were homogenized and incubated in the lysis buffer, and then centrifuged to obtain clear protein lysates. Immunodetection of proteins was performed by Western-blotting and fluorescence microscopy with specific antibodies (Figs. 9-11).

Interpretation of Figs.9: Levels of proteins caused AD are significantly increased in rats with induced disease, while they are lowered in AD rats treated with genistein. This indicates that genistein removes not only the symptoms of the disease, estimated as behavioral disturbances, but also reduces the main cause of the disease, while the correction of the behavior - shown in the specific behavioral tests - is a result of this reduction. Thus, genistein acts on the cause of the disease, not only the symptoms.

Interpretation of fig. 11: Significant increase in the number of beta-amyloid aggregates is seen in the STZ-water group thus AD rats - rats suffer from AD. On the other hand, genistein dramatically reduced number of aggregates, as demonstrated in Figs. 9-11. Thus, similar as in fig. 9, one can conclude that genistein reduced the major causes of the disease.

Biochemical tests: visualization of beta amyloid (βA) quantity in the hippocampus in the brain of rats - hippocampus in main structure responsible for memory.

## Claims

1. Genistein for use in a method of treatment of Alzheimer's disease as a stimulator of autophagy process, **characterized in that**, the genistein is used at a dose 150 mg/kg/day.

2. Genistein for use in the treatment of Alzheimer's disease according to claim 1, wherein genistein is administered orally.

3. Genistein for use in the treatment of Alzheimer's disease according to claim 1, wherein genistein is administered in form of as a powder or water suspension.

## Patentansprüche

1. Genistein zur Anwendung in einer Behandlungsmethode der Alzheimer-Krankheit als Stimulator des Autophagieprozesses, **dadurch gekennzeichnet, dass** das Genistein in einer Dosis von 150 mg/kg/Tag verwendet wird.

2. Genistein zur Verwendung bei der Behandlung der Alzheimer-Krankheit nach Anspruch 1, wobei Genistein oral verabreicht wird.

3. Genistein zur Anwendung bei der Behandlung der Alzheimer-Krankheit nach Anspruch 1, wobei Genistein in Form eines Pulvers oder einer Wassersuspension verabreicht wird.

## Revendications

1. La Génistéine pour utilisation dans le traitement de la maladie d'Alzheimer pour stimule le processus d'autophagie à une dose de 150 mg/kg/jour

2. La Génistéine pour utilisation dans le traitement la maladie d'Alzheimer selon la revendication 1, la génistéine est administrée par voie orale.

3. La Génistéine pour utilisation dans le traitement la maladie d'Alzheimer selon la revendication 1, la génistéine est administrée en poudre ou en suspension.
